(19) **Europäisches Patentamt**
**European Patent Office**
**Office européen des brevets**

(11) **EP 2 059 550 B1**

(12) **EUROPÄISCHE PATENTSCHRIFT**

(45) Veröffentlichungstag und Bekanntmachung des Hinweises auf die Patenterteilung:
**26.10.2011 Patentblatt 2011/43**

(21) Anmeldenummer: **07803000.4**

(22) Anmeldetag: **29.08.2007**

(51) Int Cl.:
*C08G 63/00* (2006.01)     *C08G 65/00* (2006.01)

(86) Internationale Anmeldenummer:
**PCT/EP2007/058983**

(87) Internationale Veröffentlichungsnummer:
**WO 2008/025794 (06.03.2008 Gazette 2008/10)**

(54) **VERFAHREN ZUR HERSTELLUNG VON POLYESTEROLEN**

METHOD FOR PRODUCING POLYESTEROLS

PROCÉDÉ DE PRÉPARATION DE POLYESTÉROLS

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **30.08.2006 EP 06119830**

(43) Veröffentlichungstag der Anmeldung:
**20.05.2009 Patentblatt 2009/21**

(73) Patentinhaber: **BASF SE**
**67056 Ludwigshafen (DE)**

(72) Erfinder:
• **GEHRINGER, Lionel**
**67630 Scheibenhard (FR)**
• **DUWENHORST, Jörn**
**49448 Lemförde (DE)**
• **HÄRING, Dietmar**
**68535 Neu-Edingen (DE)**
• **MAHN, Ulrike**
**68165 Mannheim (DE)**

• **PERETOLCHIN, Maxim**
**68161 Mannheim (DE)**
• **STUMBE, Jean-François**
**F-67200 Strasbourg (FR)**
• **KREITSCHMANN, Mirko**
**68161 Mannheim (DE)**

(56) Entgegenhaltungen:
WO-A-94/12652     WO-A-03/054204
WO-A-2006/100231

• **MATSUMURA S ET AL: "Transformation of biodegradable polyesters into oligomers under continuous flow using an enzyme-packed column" MACROMOL. BIOSCI., Nr. 4, 2004, Seiten 936-942, XP002458465**
• **MENSAH P ET AL: "Adsorptive control of water in esterification with immobilized enzymes. Continuous operation in a periodic counter-current reactor" BIOTECHNOLOGY AND BIOENGINEERING, WILEY & SONS, HOBOKEN, NJ, US, Bd. 66, 1999, Seiten 137-146, XP002444066 ISSN: 0006-3592**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft ein Verfahren gemäß Anspruch 1 zur Herstellung von Polyesterolen, die von mindestens einem Basis Polyesterol verschieden sind, aus dem mindestens einem Basis-Polyesterol und mindestens einem weiteren Reagenz, bei welchem:

    (a) das mindestens eine Basis-Polyesterol und das mindestens eine weitere Reagenz gemischt werden,

    (b) das in a) erzeugte Gemisch einen Reaktor durchströmt, wobei in dem Reaktor mindestens eine Packung mit mindestens einem immobilisierten Enzym auf einem Träger enthalten ist.

Polymere Hydroxylverbindungen wie Polyesterole und Polyetherole reagieren mit Isocyanaten zu Polyurethanen, die je nach ihren spezifischen mechanischen Eigenschaften vielfältige Einsatzmöglichkeiten finden. Insbesondere Polyesterole werden aufgrund ihrer günstigen Eigenschaften für hochwertige Polyurethanprodukte verwendet. Die spezifischen Eigenschaften der betreffenden Polyurethane hängen hierbei stark von den eingesetzten Polyesterolen ab.

Zur Herstellung von Polyurethanen ist insbesondere wichtig, dass die eingesetzten Polyesterole eine niedrige Säurezahl besitzen (siehe Ullmann's Encyclopedia, Electronic Release, Wiley-VCH-Verlag GmbH, Weinheim, 2000 unter dem Stichwort "Polyesters", Absatz 2.3 "Quality Specifications and Testing"). Die Säurezahl sollte möglichst klein sein, da die endständigen Säuregruppen langsamer mit Diisocyanaten reagieren als terminale Hydroxylgruppen. Polyesterole mit hohen Säurezahlen führen daher zu einem geringeren Molekulargewichtsaufbau während der Umsetzung von Polyesterolen mit Isocyanaten zu Polyurethan.

Ein weiteres Problem beim Einsatz von Polyesterolen mit hohen Säurezahlen zur Polyurethan-Reaktion ist, dass sich bei der Reaktion der zahlreichen endständigen Säuregruppen mit Isocyanaten eine Amidbindung unter Freisetzung von Kohlendioxid bildet. Das gasförmige Kohlendioxid kann dann zu einer unerwünschten Blasenbildung führen. Weiterhin verschlechtern freie Carboxylgruppen die Katalyse bei der Polyurethan-Reaktion und auch die Stabilität der hergestellten Polyurethane gegenüber Hydrolyse.

Nach ihrem chemischen Aufbau lassen sich die Polyesterole, d.h. Polyester mit mindestens zwei endständigen OH-Gruppen, in zwei Gruppen einteilen, die Hydroxycarbonsäure-Typen (AB-Polyester) und die Dihydroxy-Dicarbonsäure-Typen (AA-BB-Polyester). Erstere werden aus nur einem einzigen Monomer durch z.B. Polykondensation einer ω-Hydroxycarbonsäure oder durch Ringöffnungspolymerisation cyclischer Ester, sogenannter Lactone, hergestellt. Die Herstellung der AA-BB-Polyester-Typen erfolgt dagegen durch Polykondensation zweier komplementärer Monomerer, in der Regel durch die Umsetzung von mehrfunktionalen Polyhydroxylverbindungen (z.B. Diole oder Polyole) mit Dicarbonsäuren (z.B. Adipinsäure oder Terephthalsäure).

Die Polykondensation von mehrfunktionalen Polyhydroxylverbindungen und Dicarbonsäuren zu Polyesterolen des Typs AA-BB wird großtechnisch in der Regel bei hohen Temperaturen von 160 - 280°C durchgeführt. Dabei können die Polykondensationsreaktionen sowohl in Gegenwart als auch in Abwesenheit eines Lösungsmittels durchgeführt werden. Ein Nachteil dieser Polykondensationen bei hohen Temperaturen ist jedoch, dass diese verhältnismäßig langsam ablaufen. Um die Polykondensationsreaktion bei hohen Temperaturen zu beschleunigen, werden daher häufig Veresterungskatalysatoren eingesetzt. Als klassische Veresterungskatalysatoren dienen hierbei vorzugsweise metallorganische Verbindungen, wie z.B. Titantetrabutylat, Zinndioktoat oder Dibutylzinndilaurat, oder Säuren, wie z.B. Schwefelsäure, p-Toluolsulfonsäure oder Basen, wie z.B. Kaliumhydroxid oder Na-Methanolat. Diese Veresterungskatalysatoren sind homogen und verbleiben nach Beendigung der Reaktion in der Regel im Polyesterol. Nachteilig dabei ist, dass die im Polyesterol verbleibenden Veresterungskatalysatoren gegebenenfalls die spätere Umsetzung dieser Polyesterole zum Polyurethan beeinträchtigen können.

Einen weiteren Nachteil stellt die Tatsache dar, dass bei der Polykondensationreaktion bei hohen Temperaturen häufig Nebenprodukte gebildet werden. Weiterhin müssen die Hochtemperatur-Polykondensationen unter Ausschluss von Wasser stattfinden, um die Rückreaktion zu vermeiden. Dies wird in der Regel dadurch erreicht, dass die Kondensation im Vakuum, unter Inertgas-Atmosphäre oder in Anwesenheit eines Schleppgases zur vollständigen Entfernung des Wassers durchgeführt wird.

Insgesamt führen die erforderlichen Reaktionsbedingungen, insbesondere die hohen Reaktionstemperaturen, die eventuelle Inertisierung bzw. Reaktionsdurchführung im Vakuum sowie die Notwendigkeit eines Katalysators zu sehr hohen Investitions- und Betriebskosten bei der Hochtemperatur-Polykondensation.

Um diese zahlreichen Nachteile der Katalysator-unterstützten Kondensationsverfahren zu vermeiden, wurden alternative Verfahren zur Herstellung von Polyesterolen entwickelt, in denen anstelle von Veresterungskatalysatoren bei hohen Temperaturen Enzyme bei niedrigen Temperaturen verwendet werden. Als Enzyme werden hierbei in der Regel Lipasen eingesetzt, unter anderen die Lipasen *Candida antarctica, Candida cylinderacea, Mucor miehei, Pseudomonas cepacia, Pseudomonas fluorescens.*

In den bekannten Enzym-katalisierten Verfahren zur Herstellung von Polyesterolen des Typs AA-BB werden neben mehrfunktionalen Hydroxylverbindungen entweder "aktivierte Dicarbonsäure-Komponenten", z.B. in Form von Dicarbonsäure-Diestern (siehe Wallace et al., J. Polym. Sci., Part A: Polym. Chem., 27 (1989), 3271) oder "nicht aktivierte Dicarbonsäuren" verwendet. Auch diese enzymatischen Verfahren können entweder in Gegenwart oder in Abwesenheit eines Lösungsmittels durchgeführt werden.

So wird z.B. in EP 0 670 906 B1 ein Lipase-katalysiertes Verfahren zur Herstellung von Polyesterolen des Typs AA-BB bei 10 bis 90 °C offenbart, welches ohne den Einsatz eines Lösungsmittels auskommt. In diesem Verfahren können entweder aktivierte oder nicht-aktivierte Dicarbonsäure-Komponenten eingesetzt werden.

Auch in Uyama et al., Polym. J., Vol. 32, No. 5, 440 - 443 (2000) wird ein Verfahren zur Herstellung aliphatischer Polyester aus nicht-aktivierten Dicarbonsäuren und Glykolen (Sebazinsäure und 1,4-Butadiol) in einem Lösungsmittel-freien System mit Hilfe der Lipase *Candida antarctica* beschrieben.

In Binns et al, J. Polym. Sci., Part A: Polym. Chem., 36 2069 - 1080 (1998) werden Verfahren zur Herstellung von Polyesterolen aus Adipinsäure und 1,4-Butandiol mit Hilfe der immobilisierten Form der Lipase B aus *Candida antarctica* (kommerziell erhältlich als Novozym 435®) offenbart. Insbesondere wurde analysiert, welchen Einfluss die An- oder Abwesenheit eines Lösungsmittels (in diesem Fall Toluol) auf den Reaktionsmechanismus hat. Es konnte beobachtet werden, dass in Abwesenheit eines Lösungsmittels das Polyesterol im wesentlichen nur durch schrittweises Ankondensieren weiterer Monomer-Einheiten verlängert wird, während in Gegenwart von Toluol als Lösungsmittel neben der schrittweisen Bildung weiterer Veresterungen auch Umesterungsreaktionen eine Rolle spielen. Somit scheint die Enzymspezifität der eingesetzten Lipase unter anderem auch von der Gegenwart und von der Art des Lösungsmittels abzuhängen.

Den Hochtemperatur-Polykondensationen und den enzymatisch katalysierten Polykondensationen zur Herstellung von Polyesterolen ist jedoch der Nachteil gemeinsam, dass die Herstellung von Polyesterolen durch Kondensationsreaktionen in Anlagen durchgeführt wird, für die eine aufwendige Peripherie erforderlich ist. Zudem wird die Reaktion in Batch-Reaktoren durchgeführt, so dass eine kontinuierliche Herstellung der Polyesterole ebenfalls nicht möglich ist.

Bei den aus dem Stand der Technik bekannten Rührkesselreaktoren hat sich zudem gezeigt, dass hohe Katalysatorkonzentrationen, die 10 Gew.-% überschreiten, in Verbindung mit höheren Viskositäten, die mit den Polymeren einhergehen, schwierig zu handhaben sind. Insbesondere das Abfiltrieren der Enzyme aus dem Polymer zeigt sich als große technische Herausforderung, da ein hoher Druckverlust aufgrund der geringen Größe der Enzympartikel (0,3 bis 0,5 mm) erforderlich ist, weshalb höhere Drücke und demzufolge Hochdruckreaktoren notwendig werden. Höhere Scherkräfte, die aufgrund höherer Viskositäten auftreten, führen zu einer höheren Beanspruchung der immobilisierten Enzyme, die zu Abrieb führt, wodurch die Lebensdauer der Enzyme verringert wird.

Die Verwendung von kontinuierlichen Reaktoren ist aus der Herstellung von kurzkettigen Estern bekannt. Hierbei handelt es sich im Allgemeinen um Festbettreaktoren, bei denen das zur Katalyse eingesetzte Enzym auf einem Träger immobilisiert im Reaktor enthalten ist. Derartige Reaktoren werden zum Beispiel wie in P. Mensah und G. Carta, Biotechnology and Bioengineering, Vol. 66, No. 3, 1999, 137 bis 146, zur Herstellung von Isoamylpropionat und Wasser aus Propionsäure und Isoamylalkohol eingesetzt.

Eine weitere Reaktion, bei der kontinuierliche Reaktoren zur Verwendung kommen, ist die Umesterung von Geraniol mit Ethylcaproat zu Geranylcaproat. Hierbei wird ein Miniaturreaktor eingesetzt, in welchem ein auf einem Träger immobilisiertes Enzym enthalten ist. Beschrieben wird diese Reaktion von D. Pirozzi und P. J. Halling, Biotechnology and Bioengineering, Vol. 72, No. 2, 2001, 244 bis 248.

Weiterhin ist der Einsatz kontinuierlicher Reaktoren auch bekannt bei Reaktionen zum Abbau von bioabbaubaren Polyestern. Hierbei wird ein Reaktor, der eine Packung mit einem auf einem immobilisierten Träger enthaltenen Enzym enthält, eingesetzt. Das abzubauende Polymer wird zunächst in einem Lösungsmittel gelöst und anschließend durch den Reaktor geführt. Im Reaktor wird der Polyester zu cyclischen Oligomeren umgesetzt. Beschrieben wird die Reaktion von Y. Osanai et al., Macromolecular Bioscience, 2004, 4, 936 bis 942.

Allen diesen Reaktionen, bei denen ein kontinuierlicher Reaktor eingesetzt wird, ist gemeinsam, dass aus dem Reaktor ein leicht fließfähiges Gemisch austritt.

Bei der Herstellung von Polyesterolen werden jedoch hochmolekulare Reaktionsprodukte mit unterschiedlichem Molekulargewicht erzeugt, welche in Abhängigkeit von ihrer Zusammensetzung fest sein können oder eine sehr hohe Viskosität aufweisen und somit schlecht fließfähig sind.

Aufgabe der vorliegenden Erfindung ist es, ein Verfahren bereitzustellen, durch welches sich Polyesterole kontinuierlich herstellen lassen.

Gelöst wird diese Aufgabe durch ein Verfahren gemäß Anspruch 1 zur Herstellung von von den Basis-Polyesterolen verschiedenen Polyesterolen aus mindestens einem Basis-Polyesterol und mindestens einem weiteren Reagenz, bei welchem:

(a) das mindestens eine Basis-Polyesterol und das mindestens eine weitere Reagenz gemischt werden, und

(b) das in a) erzeugte Gemisch einen Reaktor durchströmt, wobei in dem Reaktor mindestens eine Packung mit mindestens einem immobilisierten Enzym auf einem Träger enthalten ist.

In dem Reaktor wird das Basis-Polyesterol durch eine enzymatisch katalysierte Umesterungsreaktion zu dem von dem Basis-Polyesterol verschiedenen Polyesterol umgesetzt.

Nach der Reaktion ist das Polyesterol insbesondere bei Prozesstemperatur flüssig. Jedoch können manche Polyesterole bei Abkühlung auskristallisieren.

Das bei der Reaktion eingesetzte Basis-Polyesterol wird zum Beispiel durch eine Polykondensation von Polyhydroxyverbindungen und Polycarbonsäuren unter Wasserabspaltung hergestellt, wobei ein Überschuss an Polyhydroxyverbindungen benötigt wird. Das Basis-Polyesterol kann hierbei zum Beispiel nach Standard-Verfahren, vorzugsweise mittels Hochtemperatur-Polykondensation, mehr bevorzugt mittels Veresterungskatalysator-unterstützter Hochtemperatur-Polykondensation hergestellt werden.

Alternativ ist es auch möglich, das Basis-Polyesterol durch eine enzymatische Polykondensation anstelle einer Veresterungskatalysator-unterstützten Hochtemperatur-Polykondensation hergestellt. Bei der enzymatischen Polykondensation wird vorzugsweise eine Lipase oder Hydrolase, vorzugsweise eine Lipase, insbesondere eine der Lipasen *Candida antarctica, Candida cylinderacea, Mucor miehei, Pseudomonas cepacia, Pseudomonas fluorescens* und *Burkholderia plantarii* bei 20 bis 120 °C, vorzugsweise bei 50 bis 90 °C, eingesetzt. Die Enzyme können hierbei auch auf einem Trägermaterial immobilisiert sein.

Wird eine Hochtemperatur-Polykondensation durchgeführt, so wird als Veresterungskatalysator vorzugsweise eine metallorganische Verbindung, zum Beispiel Titantetrabutylat, Zinndioctoat oder Dibutylzinndilaurat, oder eine Säure, zum Beispiel Schwefelsäure, p-Toluolsulfonsäure, oder eine Base, zum Beispiel Kaliumhydroxid oder Natriummethanolat, eingesetzt. Dieser Veresterungskatalysator ist in der Regel homogen und verbleibt nach Beendigung der Reaktion im Allgemeinen im Polyesterol. Die Hochtemperatur-Polykondensation wird hierbei bei 160 bis 280 °C, vorzugsweise bei 200 bis 250 °C, durchgeführt.

Bei der Herstellung des Basis-Polyesterols durch eine konventionelle Hochtemperatur-Polykondensation oder auch durch eine enzymatische Polykondensation wird das während der Kondensationsreaktion freigesetzte Wasser vorzugsweise kontinuierlich entfernt.

Als Polycarbonsäure, insbesondere Dicarbonsäure, werden vorzugsweise Adipinsäure oder andere aliphatische Dicarbonsäuren, Terephthalsäure oder andere aromatische Dicarbonsäuren eingesetzt. Als Polyhydroxylverbindung eignen sich sämtliche mindestens zweiwertigen Alkohole, jedoch vorzugsweise Diol-Komponenten wie Ethylenglykol, Diethylenglykol, 1,3-Propandiol, 1,2-Propandiol, 1,4-Butandiol, 1,5-Pentandiol, 1,6-Hexandiol, 2-Methyl-1,3-propandiol, 3-Methyl-1,5-pentandiol.

Die Polykondensation kann sowohl in Anwesenheit eines Lösungsmittels, aber auch in Abwesenheit eines Lösungsmittels, d.h. in Masse, durchgeführt werden. Dies ist davon unabhängig, ob eine (Veresterungskatalysator-unterstützte) Hochtemperatur-Polykondensation oder eine enzymatisch katalysierte Polykondensation durchgeführt wird. Die Durchführung der Polykondensation zur Herstellung des Basis-Polyesterols in Masse, d.h. in Abwesenheit jeglichen Lösungsmittels, ist jedoch bevorzugt.

Die Basis-Polyesterole werden nach den gewünschten Eigenschaften der Endprodukte ausgewählt. Bevorzugt eingesetzte Basis-Polyesterole sind Polyesterole auf Basis von Adipinsäure und einer Diol-Komponente, vorzugsweise Ethylenglykol, Diethylenglykol, 1,3-Propandiol, 1,2-Propandiol, 1,4-Butandiol, 1,5-Pentandiol, 1,6-Hexandiol, 2-Methyl-1,3-propandiol, 3-Methyl-1,5-pentandiol.

Das bevorzugte Molekulargewicht der durch die Polykondensation hergestellten Basis-Polyesterole liegt im Bereich von 200 g/mol bis 10.000 g/mol, besonders bevorzugt im Bereich von 500 bis 5000 g/mol.

Die Säurezahlen der durch die Polykondensation hergestellten Basis-Polyesterole liegen vorzugsweise im Bereich von weniger als 3 g KOH/kg, stärker bevorzugt im Bereich von weniger als 2 g KOH/kg, insbesondere im Bereich von weniger als 1 g KOH/kg. Die Säurezahl dient zur Bestimmung des Gehalts des Polyesterols an freien organischen Säuregruppen. Die Säurezahl wird ermittelt durch die Anzahl der mg KOH (bzw. g KOH), die zur Neutralisation von 1 g (bzw. 1 kg) der Probe verbraucht wird. Die Funktionalität der durch die Polykondensation hergestellten Basis-Polyesterole liegt vorzugsweise im Bereich von mindestens 1,9 bis 4,0, stärker bevorzugt im Bereich von 2,0 bis 3,0. Die Hydroxylzahl (im Folgenden als OHZ) der durch die Polykondensation hergestellten Basis-Polyesterole berechnet sich aus dem zahlenmittelgewichteten Molekulargewicht $M_n$ und der Funktionalität f des Polyesterols gemäß der Formel

$$OHZ = \frac{56100 \cdot f}{M_n}.$$

Es hat sich gezeigt, dass das erfindungsgemäße Verfahren zur Herstellung von Polyesterolen, bei dem ein Basis-Polyesterol wie vorstehend beschrieben eingesetzt wird, auch für solche Basis-Polyesterole möglich ist, die aus der klassischen Hochtemperaturkatalyse stammen, somit bereits ein relativ hohes mittleres Molekulargewicht (beispielsweise 3000 g/mol) und demzufolge auch niedrige Säurezahlen besitzen. Bislang war bekannt, dass insbesondere Polyesterole, die hohe mittlere Molekulargewichte und demzufolge niedrige Säurezahlen besitzen, nur eine sehr geringe bis gar keine Neigung zur Umesterung besitzen (siehe 2. Abschnitt von McCabe and Taylor, Tetrahedon 60 (2004), 765 bis 770).

Das weitere Reagenz, welches mit dem Basis-Polyesterol in Schritt a) gemischt wird, ist ein weiteres Polyesterol, ein Polyol, eine organische Säure oder ein Oligomer oder Polymer mit mindestens einem Hydroxyl- oder Carbonsäurerest.

Wenn das weitere Reagenz ein weiteres Polyesterol ist, so kann dieses ebenfalls wie vorstehend beschrieben hergestellt werden.

Geeignete Polyole, insbesondere Diole, die mit dem Basis-Polyesterol als weiteres Reagenz gemischt werden können, sind zum Beispiel Ethylenglykol, Diethylenglykol, 1,3-Propandiol, 1,2-Propandiol, 1,4-Butandiol, 1,5-Pentandiol, 1,6-Hexandiol, 2-Methyl-1,3-propandiol, 3-Methyl-1,5-pentandiol, Neopentylglykol, Propylenglykol, Trimethylolpropan, Pentaerythritol, Glycerin, Diglycerin, Dimethylolpropan, Di-pentaerythritol, Sorbitol, Saccharose oder andere Zucker.

Geeignete organische Säuren, die als weiteres Reagenz verwendet werden können, sind zum Beispiel Oxalsäure, Malonsäure, Bernsteinsäure, Glutarsäure, Adipinsäure, Pimelinsäure, Suberinsäure, Sebacinsäure, Maleinsäure, Ölsäure, Phthalsäure, Terephthalsäure.

Geeignete Oligomere oder Polymere mit mindestens einem Hydroxyl- oder Carbonsäurerest sind zum Beispiel Polytetrahydrofuran, Polylacton, Polyglycerole, Polyetherol, Polyesterol, $\alpha,\omega$-Dihydroxypolybutadien.

Das Gemisch, welches das Basis-Polyesterol und das mindestens eine weitere Reagenz enthält, durchströmt anschließend einen Reaktor, in dem mindestens eine Packung mit mindestens einem immobilisierten Enzym auf einem Träger enthalten ist.

Das Enzym wirkt als Katalysator für die Umsetzung des Basis-Polyesterols mit dem mindestens einen weiteren Reagenz zu dem von dem Basis-Polyesterol verschiedenen Polyesterol.

Geeignete Enzyme, die als Katalysatoren eingesetzt werden können, sind vorzugsweise Lipasen oder Hydrolasen. Bevorzugt wird eine Lipase eingesetzt, insbesondere der Lipasen *Candida antarctica, Candida cylinderacea, Mucor miehei, Pseudomonas cepacia, Pseudomonas fluorescens* und *Burkholderia plantarii.* Die Temperatur, bei der der Reaktor zur Herstellung des Polyesterols aus dem mindestens einen Basis-Polyesterol und dem mindestens einen weiteren Reagenz betrieben wird, liegt vorzugsweise im Bereich von 50 bis 110 °C, mehr bevorzugt im Bereich von 50 bis 90 °C. Der Druck, mit dem der Reaktor betrieben wird, liegt vorzugsweise im Bereich von 0,5 bis 10 bar, mehr bevorzugt im Bereich von 0,5 bis 5 bar.

Damit die Reaktion in einem kontinuierlichen Reaktor durchgeführt werden kann, ist es notwendig, dass das mindestens eine Enzym auf einem Träger immobilisiert ist. Als Trägermaterialien können alle geeigneten Materialien verwendet werden, vorzugsweise jedoch feste Materialien mit großen Oberflächen, stärker bevorzugt Harze, Polymere usw., auf denen die Enzyme vorzugsweise kovalent gebunden werden können. Geeignete Trägermaterialien sind Polyacrylat, Polyacrylamid, Polyamid, Polystyrol, Polypropylen, Polyvinylchorid, Polyurethan, Latex, Nylon, Teflon, Polypeptide, Agarose, Cellulose, Dextran, Silica, Glas, Keramik, Kieselgur, beispielsweise Celite®, Holzkohle oder Holzruß, Sägemehl, Hydroxyapatit und Aluminium. Besonders bevorzugt als Trägermaterialien sind Polyacrylat, Polyamid, Polystyrol, Silica, Glas und Keramik.

Als Trägermaterial können zum Beispiel Harzkugeln mit geringem Durchmesser (sogenannte "resin beads") verwendet werden. Derartige Harzkugeln eignen sich zum Beispiel zur Ausbildung eines Fließbettes, einer Festbettschüttung oder einer Wirbelschicht. Weiterhin ist es auch möglich, dass der Träger in Form einer Packung oder in Form von Füllkörpern vorliegt. Diese werden zum Beispiel eingesetzt, wenn der Reaktor eine strukturierte oder unstrukturierte Packung enthält, auf der das Enzym gebunden ist. Bevorzugte Träger sind Silicagel, Aluminiumoxid, Molekularsieb, anionische und kationische Ionentauscherharze.

Insbesondere bei Reaktoren, in denen das auf dem Trägermaterial immobilisierte Enzym als Fließbett, Festbett, Schüttung oder Wirbelschicht vorliegt, ist es möglich, dass durch das durchströmende Medium ein Teil des immobilisierten Enzyms aus dem Reaktor mitgerissen wird. In diesem Fall werden nach dem Durchströmen des Reaktors die auf dem Trägermaterial immobilisierten Enzyme vorzugsweise vom Polyesterol abgetrennt. Diese Abtrennung kann zum Beispiel über klassische Trennverfahren wie Filtration, Zentrifugation oder ähnliches erreicht werden, die die unterschiedliche Partikelgröße oder das unterschiedliche Partikelgewicht ausnutzen. Die Abtrennung kann beispielsweise bei magnetischen Trägermaterialien auch über die Anwendung magnetischer Kräfte erfolgen. Durch die Abtrennung der auf Trägermaterialien immobilisierten Enzyme nach dem Durchströmen des Reaktors wird verhindert, dass diese bei der Anwendung der hergestellten Polyesterole stören, so insbesondere bei weiteren Umsetzungen dieser Polyesterole wie zum Beispiel bei der Umsetzung der Polyesterole mit Isocyanaten zu Polyurethanen.

Ein zur Durchführung des Verfahrens geeigneter Reaktor umfasst vorzugsweise einen Zulauf und einen Ablauf und wird kontinuierlich von dem Reaktionsgemisch durchströmt. Im Reaktor ist mindestens eine Packung, eine Festbettschüttung oder eine Wirbelschicht enthalten, die das auf dem Träger immobilisierte Enzym enthält. Das freie Volumen der Packung, der Festbettschüttung oder der Wirbelschicht bezogen auf das Gesamtvolumen der Packung, der Festbettschüttung oder der Wirbelschicht liegt vorzugsweise im Bereich von 10 bis 100 %. Mehr bevorzugt liegt das Verhältnis des freien Volumens der Packung, der Festbettschüttung oder der Wirbelschicht bezogen auf das Gesamtvolumen der Packung, der Festbettschüttung oder der Wirbelschicht im Bereich von 30 bis 100 % und insbesondere im Bereich von 50 bis 100 %. Weiterhin liegt in der das immobilisierte Enzym enthaltenden Packung das Verhältnis des freien Strömungsquerschnitts bezogen auf den Querschnitt der Packung im Bereich von 10 bis 80 %, mehr bevorzugt im Bereich von 30 bis 78 % und insbesondere im Bereich von 50 bis 74 %.

Zur Durchführung der Reaktion des mindestens einen Basis-Polyesterols mit dem mindestens einen weiteren Reagenz ist es notwendig, dass diese gemischt werden. Dabei ist es möglich, dass das mindestens eine Basis-Polyesterol und das mindestens eine weitere Reagenz getrennt dem Reaktor zugegeben und im Reaktor gemischt werden oder aber, dass das mindestens eine Basis-Polyesterol und das mindestens eine weitere Reagenz vor Zugabe in den Reaktor gemischt werden.

Wenn das mindestens eine Basis-Polyesterol und das mindestens eine weitere Reagenz vor Zugabe in den Reaktor gemischt werden, erfolgt die Mischung vorzugsweise in einer Mischvorrichtung, wie sie dem Fachmann bekannt ist. Hierzu können zum Beispiel übliche statische oder dynamische Mischer eingesetzt werden. Derartige statische Mischer umfassen zum Beispiel Einbauten, die die Strömung umlenken und hierdurch Turbulenzen erzeugen, durch welche die Reagenzien gemischt werden. Im Unterschied dazu enthalten dynamische Mischer bewegte Teile, zum Beispiel Rotoren oder Rührer.

Das Reaktionsgemisch, wenn das mindestens eine Basis-Polyesterol und das mindestens eine weitere Reagenz vor Zugabe in den Reaktor gemischt werden, oder die Reagenzien, wenn die Mischung im Reaktor erfolgt, werden vorzugsweise am Sumpf des Reaktors zugegeben. Hierdurch wird bereits beim Anfahren, wenn der Reaktor noch nicht mit Flüssigkeit befüllt ist, eine gleichmäßige Durchströmung erzielt.

Messungen der Reaktionskinetik der enzymatischen Umesterung haben gezeigt, dass große Verweilzeiten oder hohe Katalysatorkonzentrationen notwendig sind, um die Umesterungsreaktion durchzuführen. Die für die Reaktion erforderliche Verweilzeit kann zum Beispiel dadurch erzielt werden, dass das Reaktionsgemisch den Reaktor mehrfach durchläuft. Weiterhin ist es auch möglich, die Strömungsgeschwindigkeit des Reaktionsgemisches so zu wählen, dass der Zeitraum, den das Reaktionsgemisch benötigt, um die Packung zu durchströmen, die das immobilisierte Enzym enthält, der erforderlichen Reaktionszeit entspricht.

Die Umsetzung des mindestens einen Basis-Polyesterols und des mindestens eines weiteren Reagenzes zu dem Polyesterol kann in Anwesenheit eines Lösungsmittels durchgeführt werden. Wird die Umsetzung in Anwesenheit eines Lösungsmittels durchgeführt, so können alle bekannten geeigneten Lösungsmittel eingesetzt werden, insbesondere die Lösungsmittel Toluol, Dioxan, Hexan, Tetrahydrofuran, Cyclohexan, Xylol, Dimethylsulfoxid, Dimethylformamid, N-Methylpyrrolidon, Chloroform. Die Wahl des Lösungsmittels hängt im Einzelfall von den eingesetzten Edukten (dem mindestens einen Basis-Polyesterol und dem mindestens einen weiteren Reagenz) und insbesondere von deren Löslichkeitseigenschaften ab. Die Umsetzung in Anwesenheit eines Lösungsmittels hat jedoch den Nachteil, dass zusätzliche Teilverfahrensschritte erforderlich werden, nämlich das Lösen des mindestens einen Basis-Polyesterols im Lösungsmittel und das Entfernen des Lösungsmittels nach der Umsetzung. Weiterhin kann das Lösen des mindestens einen Basis-Polyesterols im Lösungsmittel je nach den Hydrophobizitätseigenschaften des Basis-Polyesterols problematisch sein und kann gegebenenfalls die Ausbeute schmälern.

In einer bevorzugten Ausführungsform des Verfahrens erfolgt die Umsetzung des mindestens einen Basis-Polyesterols und des mindestens einen weiteren Reagenzes in Abwesenheit eines Lösungsmittels ("Umsetzung in Masse" genannt). Sollen nämlich Basis-Polyesterole mit hohem Molekulargewicht der enzymatischen Umesterung unterworfen werden, so wird die Effektivität dieser Umesterungsreaktion von der geringen Löslichkeit dieser Basis-Polyesterole mit hohem Molekulargewicht in den meisten Lösungsmitteln begrenzt. Die Anzahl der Hydroxylgruppen des Lösungsmittels hingegen hat auf die Effektivität der Umesterungsreaktion lediglich geringen Einfluss. So findet zum Beispiel nach McCabe and Taylor, Tetrahedron 60 (2004) 765 bis 770 in 1,4-Butandiol als Lösungsmittel keine Umesterungsreaktion statt, obwohl die Konzentration von Hydroxylgruppen sehr hoch ist. In polaren Lösungsmitteln (Dioxan, Toluol) findet hingegen eine Umesterung statt.

In einer weiteren bevorzugten Ausführungsform des Verfahrens werden zur Herstellung des Polyesterols vorzugsweise solche Basis-Polyesterole, Enzyme und gegebenenfalls zusätzliche Polyhydroxylverbindungen eingesetzt, die gemeinsam einen Wassergehalt von weniger als 0,1 Gew.-%, vorzugsweise von weniger als 0,05 Gew.-%, stärker bevorzugt von weniger als 0,03 Gew.-%, insbesondere von weniger als 0,01 Gew.-% besitzen. Bei höheren Wassergehalten findet während der Umsetzung des mindestens einen Basis-Polyesterols mit dem mindestens einen weiteren Reagenz neben der Umesterung auch Hydrolyse statt, so dass die Säurezahl des Polyesterols während der Umesterung in unerwünschter Weise ansteigen würde. Die Durchführung der Umesterung des erfindungsgemäßen Verfahrens bei einem Wassergehalt von weniger als 0,1 Gew.-%, vorzugsweise von weniger als 0,05 Gew.-%, stärker bevorzugt von weniger als 0,03 Gew.-%, insbesondere von weniger als 0,01 Gew.-% führt somit zur Herstellung von Spezial-Polyesterolen mit niedriger Säurezahl als Endprodukte.

Polyesterole mit niedriger Säurezahl sind im Allgemeinen hydrolysestabiler als Polyesterole mit hoher Säurezahl, da freie Säuregruppe die Rückreaktion, d.h. die Hydrolyse, katalysieren.

Eine Herstellung von Polyesterolen mit Wassergehalten von mehr als 0,1 Gew.-% führt zu Polyesterolen mit einer Säurezahl > 10 mg KOH/g. Polyesterole mit so hohen Säurezahlen (> 10 mg KOH/g) sind jedoch für die meisten industriellen Anwendungen, insbesondere zur Verwendung bei der Herstellung von Polyesterolen, nicht oder nur schlecht geeignet.

Enzyme können je nach Luftfeuchtigkeit Wassergehalte von mehr als 0,1 Gew.-% aufweisen. Deshalb ist vor dem Einsatz des Enzyms in der Umesterungsreaktion eine Trocknung des Enzyms erforderlich. Die Trocknung des Enzyms erfolgt gemäß den üblichen Trocknungsverfahren, zum Beispiel durch Trocknung in einem Vakuumtrockenschrank bei Temperaturen von 60 bis 120 °C und unter einem Druck von 0,5 bis 100 mbar oder durch Suspendieren des Enzyms in Toluol und nachfolgender Abdestillation des Toluols im Vakuum bei Temperaturen von 50 bis 100 °C.

Auch Polyesterole nehmen je nach Luftfeuchtigkeitsgehalt und Temperatur mindestens 0,01 Gew.-%, in der Regel aber mindestens 0,02 Gew.-%, in einigen Fällen auch mehr als 0,05 Gew.-% Wasser auf. Je nach Umsetzungsgrad und Molekulargewicht der eingesetzten Basis-Polyesterole ist diese Wasserkonzentration höher als die Gleichgewichts-Wasserkonzentration. Wird das Basis-Polyesterol vor der Umesterung nicht getrocknet, so setzt unweigerlich die Hydrolyse des Polyesterols ein.

Daher werden die Basis-Polyesterole, die zur Umesterung eingesetzt werden, vor der Umesterung vorzugsweise getrocknet. Auch das einzusetzende Enzym sowie das mindestens eine weitere Reagenz wird vorzugsweise vor der Umesterungsreaktion getrocknet, um den oben genannten geringen Wassergehalt bei der Umesterung zu erreichen. Die Trocknung kann anhand gängiger Trocknungsverfahren nach dem Stand der Technik durchgeführt werden, so beispielsweise durch Trocknung über Molekularsieb oder Fallfilmverdampfer. Alternativ können Basis-Polyesterole mit niedrigen Wassergehalten (vorzugsweise von weniger als 0,1 Gew.-%, bevorzugt von weniger als 0,05 Gew.-%, bevorzugter von weniger als 0,03 Gew.-%, insbesondere von weniger als 0,01 Gew.-%) auch dadurch erhalten werden, dass die Umsetzung sowie auch eventueller Schritt der Zwischenlagerung des mindestens einen Basis-Polyesterols vollständig unter inerten Bedingungen, so beispielsweise in einer Inertgas-Atmosphäre, vorzugsweise in einer Stickstoff-Atmosphäre, durchgeführt wird. In diesem Falle haben die Basis-Polyesterole von Anfang an keine Möglichkeit, größere Mengen Wasser aus der Umgebung aufzunehmen. Ein gesonderter Trocknungsschritt könnte sich dann erübrigen.

Gängige Reaktortypen, die zur Durchführung der erfindungsgemäßen Umesterung eingesetzt werden können, sind zum Beispiel Kolonnen, die eine strukturierte oder unstrukturierte Packung enthalten, Fließbettreaktoren oder Wirbelschichtreaktoren. Das Material, aus welchem der Reaktor ausgeführt ist, muss sowohl korrosionsbeständig, hitze- als auch säurebeständig sein. Geeignete Materialien sind zum Beispiel Edelstahl, Glas oder Keramik. Geeignete Edelstahle sind zum Beispiel austenitische Chrom-Nickel-Molybdän-Legierungen (zum Beispiel V4A-Stahl DIN 1.4571).

Im Folgenden wird die Erfindung anhand eines Beispiels näher beschrieben.

## Beispiel

Das Basis-Polyesterol Polyethylenglykoladipinat wird in einem geheizten Rührkessel vorgelegt. Diesem wird unter Rühren Diethylenglykol zugegeben, um die gewünschte Säurezahl von 150 mg KOH/g zu erhalten. Anschließend wird das Gemisch einer Reaktionskolonne zugegeben, die eine Packung aus Novozym 435® enthält. Hierbei handelt es sich um die kommerziell erhältliche, immobilisierte Form der Lipase B aus *candida antarctica.* Im Reaktor wird das im Reaktor durch Umesterung erhaltene Reaktionsprodukt in einen Auffangbehälter geleitet. In regelmäßigen Zeitintervallen werden Proben zur Bestimmung der Viskosität und der GPC der Kolonne entnommen. Die Durchflussraten liegen im Bereich von 860 bis 1000 g/h. Um die Lebensdauer des Enzyms zu prüfen, wird eine Gesamtmenge von 95 kg des Gemisches aus Polydiethylenglykoladipinat und Diethylenglykol der Kolonne über einen Zeitraum von 5 Tagen zugeführt.

Die eingesetzte Kolonne weist einen Durchmesser von 30 mm und eine Länge von 1 m auf und ist aus Glas gefertigt. Das Volumen der Kolonne beträgt 700 ml. Die Kolonne ist mit 180 g des getrockneten Enzyms, das in Polyesterol gelöst wurde, befüllt. Die Konzentration an Novozym® liegt bei 25 Gew.-%. Eine höhere Füllung ist nicht möglich aufgrund der Quellung des Katalysators und dem daraus resultierenden steigenden Druckverlust.

## Patentansprüche

1. Verfahren zur Herstellung von Polyesterolen, die von mindestens einem Basis-Polyesterol verschieden sind, aus dem mindestens einem Basis-Polyesterol und mindestens einem weiterem Reagenz, wobei das weitere Reagenz ein Polyesterol, ein Polyol, eine organische Säure oder ein Oligomer oder Polymer mit mindestens einem Hydroxyl- oder Carbonsäurerest ist und das Verfahren kontinuierlich durchgeführt wird und bei welchem

    (a) das mindestens eine Basis-Polyesterol und das mindestens eine weitere Reagenz gemischt werden,
    (b) das in (a) erzeugte Gemisch einen Reaktor durchströmt, wobei in dem Reaktor mindestens eine Packung mit mindestens einem immobilisierten Enzym auf einem Träger enthalten ist, wobei das mindestens eine Basis-Polyesterol und das mindestens eine weitere Reagenz zu dem Polyesterol durch eine enzymatisch katalysierte Umesterungsreaktion umgesetzt werden, wobei in der das immobilisierte Enzym enthaltenden Packung das Verhältnis des freien Strömungsquerschnitts bezogen auf den Querschnitt der Packung im Bereich von 10 bis 80 % liegt.

**2.** Verfahren nach Anspruch 1, **dadurch gekennzeichnet, dass** das mindestens eine Basis-Polyesterol und das mindestens eine weitere Reagenz getrennt dem Reaktor zugegeben und im Reaktor gemischt werden.

**3.** Verfahren nach Anspruch 1, **dadurch gekennzeichnet, dass** das mindestens eine Basis-Polyesterol und das mindestens eine weitere Reagenz vor Zugabe in den Reaktor gemischt werden.

**4.** Verfahren nach Anspruch 1, **dadurch gekennzeichnet, dass** der Reaktor mit einer Temperatur im Bereich von 50 bis 120°C und einem Druck im Bereich von 0,5 bis 10 bar betrieben wird.

**5.** Verfahren nach Anspruch 1, **dadurch gekennzeichnet, dass** das mindestens eine Enzym eine Lipase oder eine Hydrolase ist.

**6.** Verfahren nach Anspruch 5, **dadurch gekennzeichnet, dass** die Lipase ausgewählt ist aus *Candida antarctica, Candida cylinderacea, Mucor miehei, Pseudomonas cepacia, Pseudomonas fluorescens* und *Burkholderia plantarii.*

**Claims**

**1.** A process for preparing polyesterols which are different from at least one base polyesterol from the at least one base polyesterol and at least one further reagent, wherein the further reagent is a polyesterol, a polyol, an organic acid or an oligomer or polymer having at least one hydroxyl or carboxylic acid radical and the process is carried out continuously and

(a) the at least one base polyesterol and the at least one further reagent are mixed,
(b) the mixture produced in (a) flows through a reactor in which at least one packing comprising at least one immobilized enzyme on a support is present, with the at least one base polyesterol and the at least one further reagent being reacted by means of an enzymatically catalyzed transesterification reaction to form the polyesterol, where, in the packing comprising the immobilized enzyme, the ratio of the free flow cross section to the cross section of the packing is in the range from 10 to 80%.

**2.** The process according to claim 1, wherein the at least one base polyesterol and the at least one further reagent are introduced separately into the reactor and mixed in the reactor.

**3.** The process according to claim 1, wherein the at least one base polyesterol and the at least one further reagent are mixed before introduction into the reactor.

**4.** The process according to claim 1, wherein the reactor is operated at a temperature in the range from 50 to 120°C and a pressure in the range from 0.5 to 10 bar.

**5.** The process according to claim 1, wherein the at least one enzyme is a lipase or a hydrolase.

**6.** The process according to claim 5, wherein the lipase is selected from among *Candida antarctica, Candida cylinderacea, Mucor miehei, Pseudomonas cepacia, Pseudomonas fluorescens* and *Burkholderia plantarii.*

**Revendications**

**1.** Procédé de préparation de polyestérols qui sont différents d'au moins un polyestérol de base, à partir du ou des polyestérols de base et d'au moins un autre réactif, l'autre réactif étant un polyestérol, un polyol, un acide organique ou un oligomère ou un polymère comportant au moins un radical hydroxyle ou acide carboxylique, le procédé étant mis en oeuvre en continu, procédé dans lequel

(a) on mélange le ou les polyestérols de base et le ou les autres réactifs,
(b) on fait passer dans un réacteur le mélange produit en (a), le réacteur contenant au moins un garnissage constitué d'au moins une enzyme immobilisée sur un support, le ou les polyestérols de base et le ou les autres réactifs étant mis à réagir pour donner le polyestérol, par une réaction de transestérification à catalyse enzymatique, le rapport, dans le garnissage contenant l'enzyme immobilisée, entre la section transversale libre d'écoulement et la section transversale du garnissage, étant compris dans la plage de 10 à 80 %.

**2.** Procédé selon la revendication 1, **caractérisé en ce que** le ou les polyestérols de base et le ou les autres réactifs sont introduits séparément dans le réacteur et sont soumis à un mélange dans le réacteur.

**3.** Procédé selon la revendication 1, **caractérisé en ce que** le ou les polyestérols de base et le ou les autres réactifs sont mélangés avant leur introduction dans le réacteur.

**4.** Procédé selon la revendication 1, **caractérisé en ce que** le réacteur est exploité à une température comprise dans la plage de 50 à 120°C et sous une pression comprise dans la plage de 0,5 à 10 bar.

**5.** Procédé selon la revendication 1, **caractérisé en ce que** la ou les enzymes sont des lipases ou des hydrolases.

**6.** Procédé selon la revendication 5, **caractérisé en ce que** la lipase est choisie parmi *Candida antarctica, Candida cylinderacea, Mucor miehei, Pseudomonas cepacia, Pseudomonas fluorescens* et *Burkholderia plantarii.*

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0670906 B1

**In der Beschreibung aufgeführte Nicht-Patentliteratur**

- Ullmann's Encyclopedia, Electronic Release. Wiley-VCH-Verlag GmbH, 2000
- **WALLACE et al.** *J. Polym. Sci., Part A: Polym. Chem.,* 1989, vol. 27, 3271
- **UYAMA et al.** *Polym. J.,* 2000, vol. 32 (5), 440-443
- **BINNS et al.** *J. Polym. Sci., Part A: Polym. Chem.,* 1998, vol. 36, 2069-1080
- **P. MENSAH ; G. CARTA.** *Biotechnology and Bioengineering,* 1999, vol. 66 (3), 137-146
- **D. PIROZZI ; P. J. HALLING.** *Biotechnology and Bioengineering,* 2001, vol. 72 (2), 244-248
- **Y. OSANAI et al.** *Macromolecular Bioscience,* 2004, vol. 4, 936-942
- **MCCABE ; TAYLOR.** *Tetrahedon,* 2004, vol. 60, 765-770
- **MCCABE ; TAYLOR.** *Tetrahedron,* 2004, vol. 60, 765-770